# EUROPEAN PATENT SPECIFICATION

(11) **EP 2 409 147 B1**
(45) Date of publication and mention of the grant of the patent: **30.07.2014**
(21) Application number: 10714712.6
(22) Date of filing: 16.03.2010
(51) Int. Cl.: G01N 33/34, G01N 21/64, G01N 21/85

(54) **USE OF HYDROPHOBIC DYES TO MONITOR HYDROPHOBIC CONTAMINANTS IN A PAPERMAKING PROCESS**
VERWENDUNG HYDROPHOBER FARBSTOFFE ZUR KONTROLLE HYDROPHOBER VERUNREINIGUNGEN BEI EINEM PAPIERHERSTELLUNGSVERFAHREN
UTILISATION DE COLORANTS HYDROPHOBES POUR SURVEILLER DES CONTAMINANTS HYDROPHOBES DANS UN PROCESSUS DE FABRICATION DE PAPIER

(30) Priority: 17.03.2009 US 405807
(43) Date of publication of application: 25.01.2012
(73) Proprietor: Nalco Company, Naperville, IL 60563-1198 (US)
(72) Inventor: GERLI, Alessandra, NL-2313 GD Leiden (NL); SHERMAN, Laura M., Naperville, Illinois 60565 (US); MURCIA, Michael J., Dekalb, Illinois 60115 (US)
(74) Representative: HGF Limited
(86) International application number: PCT/US2010/027380
(87) International publication number: WO 2010/107725

(56) References cited:
- WO-A1-2007/082376
- WO-A1-2008/104576
- WO-A1-2008/144383

## Description

### FIELD OF THE INVENTION

This invention pertains to the measurement and control of hydrophobic contaminants.

### BACKGROUND OF THE INVENTION

Hydrophobic/organic contaminants, such as natural pitch, stickies, tackies and white pitch are major obstacles in paper manufacturing because these materials when liberated during a papermaking process can become both undesirable components of papermaking furnishes and troublesome to the mill equipment by preventing proper operation of mechanical parts when these materials deposit

Increased use of secondary fiber, coated broke and mechanical pulp in the papermaking process contributes to the accumulation of organic contaminants. These contaminants can form deposits that affect machine runnability and final product quality. Control of the contaminants is typically managed through chemical fixation, and its effectiveness is therefore dependent on the ability to determine the proper program and application. Historically, a common method used to assess program performance has been filtrate turbidity reduction. This method, however, is not entirely adequate because it often yields an incomplete picture of the furnish demands from hydrophobic particles. More recently, flow cytometry has been used in the industry for monitoring hydrophobic contaminants. Disadvantages of this method are that it is both labor and capital intensive.

A rapid and accurate method of measuring organic contaminants is therefore desired. Chemical control programs are often used to passivate or remove deposit-forming contaminants. For that reason, a method of screening the efficacy of chemical treatments that reduce the overall content of hydrophobic contaminants in a papermaking process is also desired.

WO 2008/104576 is concerned with a method for determining the concentration of hydrophobic organic particles in the filtrate of a paper fabric.

WO 2007/082376 and WO 2008/144383 discusse methods for measuring hydrophobic contaminants distributed in paper pulp suspension.

### BRIEF DESCRIPTION OF THE DRAWINGS

Figure 1 shows pulp samples collected from various locations across the wet end of a paper machine as indicated. The results indicate that turbidity and hydrophobicity, or contaminant presence, do not necessarily track each other.
Figure 2 shows the hydrophobicity of untreated and treated coated broke filtrate filtered through various size media of 0.8, 3, 5, 10, and 76 microns.

Figure 3 shows that different fixatives respond to the coated broke differently. Although different fixatives can achieve the same turbidity, the hydrophobicity varies greatly among the samples.

### SUMMARY OF THE INVENTION

The present invention provides for a method of monitoring one or more types of hydrophobic contaminants in a papermaking process comprising: (a) obtaining a sample of fluid from said papermaking process; (b) measuring the turbidity of the sample of fluid. (c) selecting a hydrophobic dye that is capable of interacting with said contaminants in said fluid and fluorescing in said fluid; (d) adding said dye to said fluid and allowing a sufficient amount of time for said dye to interact with said contaminants in said fluid; (e) measuring the fluorescence of the dye in said fluid; (f) correlating the fluorescence of the dye with the concentration or said contaminants; and wherein if the turbidity as measured in step (b) is above 2000NTU then said sample is diluted further separated prior to addition of said dye in step (d) and fluorescent measurement.

The present invention may further comprise measuring the effectiveness of one or more chemicals that decrease the amount of one or more hydrophobic contaminants in a papermaking process: (a) monitoring one or more types of contaminants in a papermaking process comprising: obtaining a sample of fluid from said papermaking process; selecting a hydrophobic dye that is capable of interacting with said contaminants in said fluid and fluorescing in said fluid; adding said dye to said fluid and allowing a sufficient amount of time for said dye to interact with said contaminants in said fluid; measuring the fluorescence of the dye in said fluid; and correlating the fluorescence of the dye with the concentration of said contaminants; (b) adding one or more chemicals to said papermaking process that decrease the amount of said hydrophobic contaminants in said papermaking process; (c) re-measuring the amount of contaminants in said papermaking process by performing step (a) at least one more time; and (d) optionally controlling the amount of said chemicals that are added to said papermaking process.

The present invention may further comprise monitoring one or more hydrophobic contaminants and determining the size of said hydrophobic contaminants in a papermaking process comprising: (a) obtaining a sample of fluid from said papermaking process; (b) selecting a hydrophobic dye that is capable of interacting with said contaminants in said fluid and fluorescing in said fluid; (c) adding said dye to said fluid and allowing a sufficient amount of time for said dye to interact with said contaminants in said fluid; (d) measuring the fluorescence of the dye in said fluid; (e) correlating the fluorescence of the dye with the concentration of said contaminants; (f) passing the sample measured in step (d) through a medium capable of separating the sample into one or more aqueous fractions at least one time; (g) measuring the fluorescence of the aqueous fractions from step (f) of said sample at least one time; (h) determining the size of the hydrophobic contaminants of the aqueous fractions; (i) optionally correlating the fluorescence of the dye in the aqueous fractions with the concentration of said hydrophobic contaminants in the aqueous fractions; and optionally controlling the amount of one or more chemicals that reduce or inactivate said contaminants which are added to said papermaking process.

The present invention may further comprise monitoring one or more hydrophobic contaminants and determining the size of said hydrophobic contaminants in a papermaking process comprising: (a) obtaining a sample of fluid from said papermaking process; (b) selecting a hydrophobic dye that is capable of interacting with said contaminants in said fluid and fluorescing in said fluid; (c) adding said dye to said fluid and allowing a sufficient amount of time for said dye to interact with said contaminants in said fluid; (d) measuring the fluorescence of the dye in said fluid; (e) correlating the fluorescence of the dye with the concentration of said contaminants; (f) filtering the sample measured in step (d) at least one time through at least one filter, wherein the filter has one or more pores with a known pore size; (g) measuring the fluorescence of the filtrate from step (f) of said sample at least one time; (h) determining the size of the hydrophobic contaminants of the filtrate and optionally a concentrate from said filtration step; (i) optionally correlating the fluorescence of the dye in the filtrate and/or reject with the concentration of said hydrophobic contaminants in the filtrate; and
(j) optionally controlling the amount of one or more chemicals that reduce or inactivate said contaminants which are added to said papermaking process.

### DETAILED DESCRIPTION OF THE INVENTION

"Papermaking process" means a method of making any kind of paper products (e.g. paper, tissue, board, etc.) from pulp comprising forming an aqueous cellulosic papermaking furnish, draining the furnish to form a sheet and drying the sheet. The steps of forming the papermaking furnish, draining and drying may be carried out in any manner generally known to those skilled in the art. The papermaking process may include a pulping stage, e.g. making pulp from woody raw material and bleaching stage, e.g. chemical treatment of the pulp for brightness improvement. Furnishes can contain fillers and/or other contaminants.

"Bulk sample" means a sample whose constituents have not been specifically separated, except bulk sample may include, a separation based upon size. For example, bulk sample does not include separating e.g. a resin particle from a suspension.

"Solvatochromatic dye" is a dye that has a shifting absorbance and/or fluorescence emission wavelength depending on the polarity of its surroundings.

"Fluid" includes an aqueous papermaking suspension from a papermaking process, e.g. a fluid containing fibers in a pulping stage, a thin stock, a thick stock, aqueous suspensions drawn from the papermaking process, e.g. various locations from a papermaking machine or pulping process, aqueous fluid in a uhl box, press dewatering section, and/or any part of the papermaking process that one of ordinary skill in the art can think of where one would need to monitor hydrophobic contaminants.

As stated above, the present invention provides for a method of monitoring one or more types of hydrophobic contaminants in a papermaking process via the use of fluorescence.

The hydrophobic dyes, which are added to the sample, must be able to stain or interact with the hydrophobic contaminants, e.g. pitch particles.

In one embodiment, a method of monitoring hydrophobic dyes consists essentially of the above stated elements.

In another embodiment, the fluid is an aqueous filtrate of a pulp slurry.

According to the invention, the turbidity of the fluid is also measured. In a further embodiment, the turbidity of said fluid is measured before and after the addition of said chemicals.

According to the invention, the fluid is filtered or diluted or a combination thereof prior to said addition of said dye or said fluorescent measurement of said dye, wherein said filtering or dilution of said fluid permits said fluid to be fluorometrically monitored.

In another embodiment, the sample is taken from a dilute sample point off a papermaking process, e.g. a paper machine. In a further embodiment, the sample point is the white water of a papermaking process. The reasoning postulated for this collection/sample point is that there is no long fiber present/substantially any fiber present, and filtration may not be necessary.

In another embodiment, one or more samples undergo a sieving/separation step to separate the long fiber from the suspended contaminants in a sample solution. For example, the degree of dilution that the filtrate/aqueous fraction undergoes from the separation process relies on two main factors, both relating to turbidity. If the filtrate/aqueous fraction is too turbid for the turbidimeter, dilution is required to bring the turbidity into a measurable range for the meter. This is the case unless you want a less accurate and "quick and dirty" test, which is one embodiment of the claimed invention.

According to the invention if the turbidity is above 2000 NTU (nephelometric turbidity units), then said sample from a papermaking process is diluted/further separated prior to the addition of a dye and fluorescent measurement. The value of 2000 NTU may be instrument or measurement technique dependent.

In another embodiment, the dye is selected from the group consisting of: 9-diethylamino-5H-benzo[alpha]phenoxazine-5-one, 1-dimethylamino-5-sulfamoyl-naphthalene, pyrene, 1-pyrenecarbaldehyde, Reichardt's dye, 4-aminophthalimide, 4-(N,N-dimethylamino)phthalimide, bromonapthalene, 2-(dimethylamino)naphthalene, and a combination thereof.

In another emebodiment, the dye is a solvatochromatic dye.

In another embodiment, the dye does not include N-(n-butyl)-4-(n-butylamino)-naphthalimide.

In another embodiment, the fluid is obtained from a wet end of said papermaking process.

As stated above, the dye added to a sample must have a sufficient amount of time for said dye to interact with said contaminants in said fluid prior to its fluorescent measurement. One of ordinary skill in the art could determine a sufficient amount of time for said interaction without undue experimentation.

In one embodiment, the dye is mixed with a solvent prior to its addition to said fluid. One of ordinary skill in the art could determine an adequate time for mixing without undue experimentation.

In another embodiment, the contaminants are selected from the group consisting of: pitch, fiber, filler, fines, coated broke, mill broke, recycle, groundwood, thermal mechanical pulp, chemi-thermal mechanical pulp, chemical pulp, deinked pulp, ink, adhesives, stickies, tackies, waxes, binders and dissolved and/or colloidal substances, and a combination thereof.

In another embodiment, the method is an on-line method and/or batch sample method.

In another embodiment, the fluorometric measurement is performed at a pre-set basis, intermittent basis, and/or continuous basis. For example, a flow cell can be utilized as a means for measuring the fluorescence of said hydrophobic contaminants. More specifically, in one embodiment, a process for measurement comprises: the addition of one or more fluorescent tracers to a sample obtained from a papermaking process prior to its fluorescent measurement in said flow cell. One of ordinary skill in the art would be able to carry out this process without undue experimentation. For example, one could utilize flow injection analysis and/or sequence injection analysis techniques to carry out the above-referenced measurement protocol.

In another embodiment, the fluorometric measurement is performed with a handheld fluorometer. A fluorescent measurement may be carried out with other types of fluorometers.

The present invention also provides for measuring the effectiveness of one or more chemicals that decrease the amount of one or more hydrophobic contaminants in a papermaking process. The information on the amount of hydrophobic contaminants in a fluid can be utilized to form a control loop for the addition of one or more chemicals, which can be used to control the amount of hydrophobic contaminants.

In one embodiment, the methodology for monitoring the hydrophobic contaminants can be measured by the above-stated fluorescence methodology and its various embodiments.

In another embodiment, a determination of the amount of fluorescence is measured by the above-mentioned protocol, then subsequent to this step, an addition of one or more chemicals to the papermaking process to treat the hydrophobic contaminants, e.g. increase/decrease in the same chemistry for hydrophobic contaminant inhibition or change in the chemistry treatment program for hydrophobic contaminant inhibition, and then subsequent to the treatment step, a re-measurement of the amount of contaminants in said papermaking process by the above-mentioned protocol.

In another embodiment, the chemicals are at least one of the following: a fixative; a detackifier; a dispersant; a surfactant; and a retention aid.

The present invention also provides for monitoring hydrophobic contaminants and determining the size of said hydrophobic contaminants in a papermaking process.

In one embodiment, the methodology for monitoring the hydrophobic contaminants can be measured by the above-stated fluorescence methodology and its various embodiments.

In anther embodiment, the aqueous fractions contain one or more suspended solids or one or more particles.

In another embodiment, the medium capable of separating is a centrifuge, a filter or a combination thereof.

Various types of apparatuses/techniques, filters can be utilized, e.g. with various pore sizes, can be utilized to separate components based upon size.

### EXAMPLE SECTION

### A. Standard Testing Procedure

### FLUORESCENCE MEASUREMENT OF COATED BROKE FILTRATE

I. Equipment: Turner Designs Aquafluor® fluorometer unit with green optic channel (A) was utilized.
II. Calibration: The Aquafluor fluorometer unit is calibrated by measuring the fluorescence value of a solution containing 30 ppm of nile red dye in alcohol (3 microliters of the 0.01wt% nile red dye solution in 3 mls of alcohol). This fluorescence emission is set to 600 units on the instrument and the fluorescence emission of de-ionized water was measured and used as the 'blank'.
III. Coated Broke Treatment: After performing the coated broke treatment test as described in the table, (the Britt jar propeller is used for mixing the broke) the filtrate is diluted to obtain an on-scale turbidity reading.
Coated Broke Treatment Test Parameters

| Timing Sequence | |
|---|---|
| t = 0 | Start |
| t = 10 | Add Coagulant |
| t = 30 | Stop & Filter |
| Collect filtrate for 60 seconds and record mass | |
| Settings | |
| 150 g Sample @ ∼3.5 wt% | |
| 500 rpm | |
| 100 mesh screen (3" dia) | |
| Make appropriate dilution of the filtrate for turbidity reading | |

IV. Fluorescence Measurement:
1. After the turbidity of the dilute solution is read, place 3 mls of dilute coated broke filtrate into disposable cuvette and turn Aquafluor unit on.
2. Wipe exterior of cuvette with non-abrasive wipe, place cuvette into Aquafluor unit, close lid, press the read button, and record the background fluorescence.
3. Add 6 microliters of 0.01 % nile red dye solution in alcohol to cuvette. Mix well. Wipe exterior of cuvette with non-abrasive wipe.
4. Place cuvette into Aquafluor unit, close lid, press the read button, and record the raw fluorescence measurement result.
5. When finished, discard sample. Throw out cuvette if unable to wash out.
6. Repeat for all samples.
7. Subtract the background fluorescence from the sample fluorescence to obtain the corrected fluorescence.

V. Data Analysis : The data can be plotted various ways including:
1. % Reduction in Hydrophobicity (fluorescence reduction) vs. polymer dose;
2. Turbidity vs. fluorescence for each dosage curve

### B. Examples

The following examples utilize the above referenced "Standard Testing Procedure" for fluorescence measurements.

### Example 1

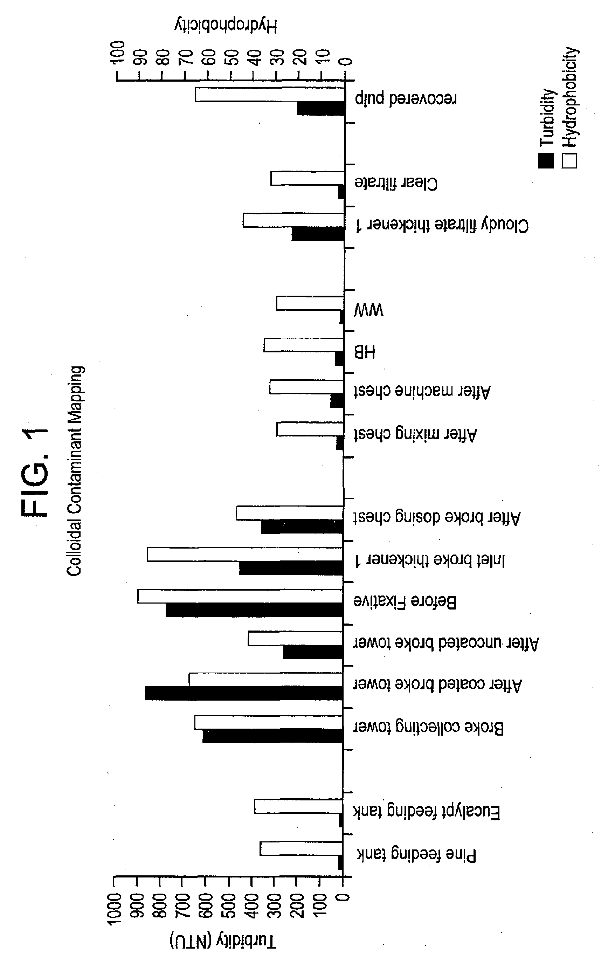

Pulp samples were collected from various locations across the wet end of the paper machine as indicated in the figure. The long fibers were removed from the sample by passing the sample through a 150-micron sieve. Turbidity of the filtrate was measured, followed by a fluorescence measurement on the filtrate after adding 6 microliters of 0.01 wt% nile red dye in alcohol to 3 milliliters of the filtrate. The results, as shown in Figure 1, indicate that turbidity and hydrophobicity, or contaminant presence do not necessarily track each other.

### Example 2

Preparation of coated broke: The coated broke was prepared from dry coated paper cut into 1.5 by 1.5 inch squares. 140 g of dry coated broke was soaked for 20 minutes in 3,860 ml of synthetic tap water for a target consistency of 3.5 wt%. The sample was then transferred to a four-liter capacity Adirondack Pulper and repulped at setting 5 for 90 minutes.

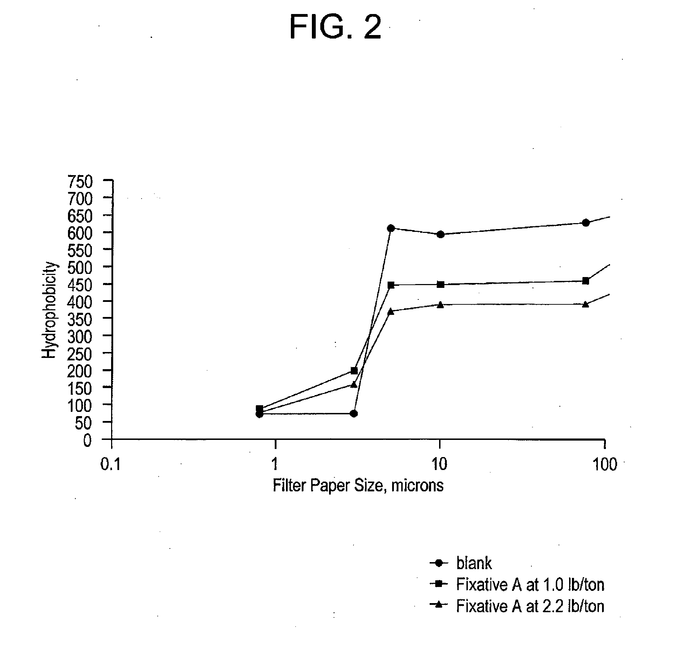

Figure 2 shows the hydrophobicity of untreated and treated coated broke filtrate filtered through various size media of 0.8, 3, 5, 10, and 76 microns. The fluorescence measured on each of the filtrates provides an indication of which particle size region contains the highest level of hydrophobic particles. The figure also indicates that there is no agglomeration of the white pitch upon addition of fixative. The fixative lowers the overall hydrophobicity while maintaining the particle size distribution profile.

### Example 3

This hydrophobicity test method was used to evaluate potential treatment programs for a coated broke sample.

### Test Procedure:

1. Prepare fixative solutions at 0.3 to 0.5 weight-percent as product.
2. Set mixing Britt jar mixing at 500 rpm
3. Add 200 mL of coated broke to the 400 mL-beaker.
4. Insert Britt Jar propeller into broke, turn Britt Jar mixer on, and at the same time start stop watch.
5. Add coagulant using the following mixing sequence:
   t = 0, start mixer
   t = 10 seconds, add coagulant
   t = 30 seconds, stop mixer, stop and reset stopwatch.
6. Pour the whole treated stock into the 100-mesh sieve supported on a 400 mL beaker and start stop watch. When filtration is completed, remove sieve from beaker and monitor time of filtration.
7. Measure turbidity of the filtrate by using a turbidity meter. If the filtrate turbidity is out of range, dilute filtrate. The same dilution must be applied to each of the filtrates from the polymer-dosed stocks in order to evaluate relative polymer performance.
8. Take 3 ml of diluted filtrate and add 3 microliters of 0.01 wt% nile red dye in alcohol and measure the fluorescence.
9. Repeat steps 3 through 8 with no polymer treatment (blank).

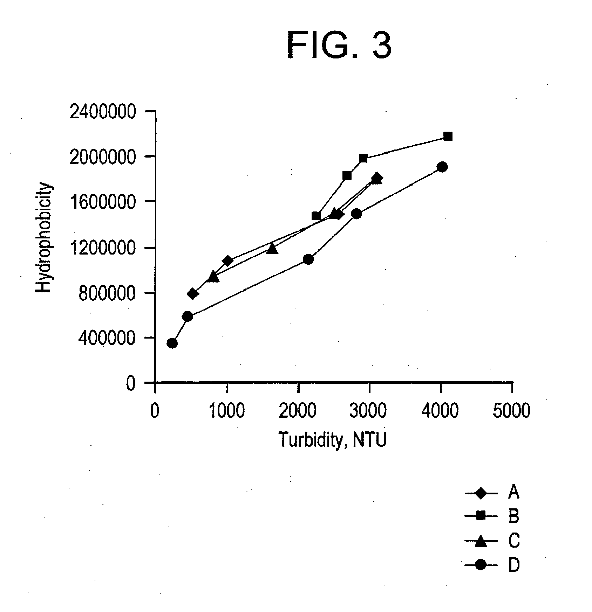

Figure 3 demonstrates that different fixatives respond to the coated broke differently. Although the same turbidity can be achieved by different fixatives, the hydrophobicity varies greatly among the samples.

## Claims

1. A method of monitoring one or more types of hydrophobic contaminants in a papermaking process comprising:
(a) obtaining a sample of fluid from said papermaking process;
(b) measuring the turbidity of the sample of fluid;
(c) selecting a hydrophobic dye that is capable of interacting with said contaminants in said fluid and fluorescing in said fluid;
(d) adding said dye to said fluid and allowing a sufficient amount of time for said dye to interact with said contaminants in said fluid;
(e) measuring the fluorescence of the dye in said fluid;
(f) correlating the fluorescence of the dye with the concentration of said contaminants; and
wherein if the turbidity as measured in step (b) is above 2000 NTU then said sample is diluted / further separated prior to addition of said dye in step (d) and fluorescent measurement.

2. The method of claim 1, wherein said fluid is filtered or diluted or a combination thereof prior to said addition of said dye or said fluorescent measurement of said dye.

3. The method of claim 1, wherein said dye is selected from the group consisting of: 9-diethylamino-5H-benzo[alpha]phenoxazine-5-one, 1-dimethylamino-5-sulfamoyl-naphthalene, pyrene, 1-pyrenecarbaldehyde, Reichardt's dye, 4-aminophthalimide, 4-(N,N-dimethylamino)phthalimide, bromonapthalene, 2-(dimethylamino)naphthalene, and a combination thereof.

4. The method of claim 1, wherein said dye does not include N-(n-butyl)-4-(n-butylamino)-naphthalimide.

5. The method of claim 1, wherein said dye is a solvatochromatic dye.

6. The method of claim 1 wherein said fluid is obtained from a wet end of said papermaking process.

7. The method of claim 1, wherein said fluorometric measurement is performed at a pre-set basis, intermittent basis, and/or continuous.

8. The method of claim 1, wherein said fluid is an aqueous filtrate of a pulp slurry.

9. The method of claim 1, wherein the method is for measuring the effectiveness of one or more chemicals that decrease the amount of one or more hydrophobic contaminants in a papermaking process, the method further comprising:
(i) adding one or more chemicals to said papermaking process that decrease the amount of said hydrophobic contaminants in said papermaking process;
(ii) re-measuring the amount of contaminants in said papermaking process by performing steps (a) to (f) at least one more time; and
(iii) optionally controlling the amount of said chemicals that are added to said papermaking process.

10. The method of claim 9, wherein said chemicals are at least one of the following: a fixative; a detackifier; a dispersant; a surfactant; and a retention aid.

11. The method of claim 1, wherein the method also determines the size of said hydrophobic contaminants in a papermaking process, the method further comprising:
(g) passing the sample measured in step (e) through a medium capable of separating the sample into one or more aqueous fractions at least one time;
(h) measuring the fluorescence of the aqueous fractions from step (g) of said sample at least one time;
(i) determining the size of the hydrophobic contaminants of the aqueous fractions;
(j) optionally correlating the fluorescence of the dye in the aqueous fractions with the concentration of said hydrophobic contaminants in the aqueous fractions; and
(k) optionally controlling the amount of one or more chemicals that reduce or inactivate said contaminants which are added to said papermaking process.

12. The method of claim 11, wherein said aqueous fractions contain one or more suspended solids or one or more particles.

13. The method of claim 11, wherein the medium capable of separating is a centrifuge, a filter or a combination thereof.

14. The method of claim 1, wherein the method also determines the size of said hydrophobic contaminants in a papermaking process, the method further comprising:
(g) filtering the sample measured in step (e) at least one time through at least one filter, wherein the filter has one or more pores with a known pore size;
(h) measuring the fluorescence of the filtrate from step (g) of said sample at least one time;
(i) determining the size of the hydrophobic contaminants of the filtrate and optionally a concentrate from said filtration step;
(j) optionally correlating the fluorescence of the dye in the filtrate and/or reject with the concentration of said hydrophobic contaminants in the filtrate; and
(k) optionally controlling the amount of one or more chemicals that reduce or inactivate said contaminants which are added to said papermaking process.

## Patentansprüche

1. Verfahren zur Überwachung einer oder mehrerer Arten von hydrophoben Verunreinigungen in einem Papierherstellungsverfahren, umfassend:
(a) Nehmen einer Fluidprobe aus dem Papierherstellungsverfahren;
(b) Messen der Trübheit der Fluidprobe;
(c) Auswählen eines hydrophoben Farbstoffs, der in der Lage ist, mit den Verunreinigungen in dem Fluid zu reagieren und in dem Fluid zu fluoreszieren;
(d) Zugeben des Farbstoffs zu dem Fluid und Warten, bis ausreichend Zeit vergangen ist, damit der Farbstoff mit den Verunreinigungen im Fluid wechselwirken konnte;
(e) Messen der Fluoreszenz des Farbstoffs in dem Fluid;
(f) Korrelieren der Fluoreszenz des Farbstoffs mit der Konzentration der Verunreinigungen; und
wobei in dem Fall, dass die Trübheit, die im obigen Schritt (b) gemessen wird, oberhalb von 2000 NTU liegt, die Probe verdünnt wird / weiter getrennt wird, bevor in Schritt (d) der Farbstoff zugegeben wird und die Fluoreszenz gemessen wird.

2. Verfahren nach Anspruch 1, wobei das Fluid vor der Zugabe des Farbstoffs oder der Messung der Fluoreszenz des Farbstoffs gefiltert oder verdünnt wird oder einer Kombination davon unterzogen wird.

3. Verfahren nach Anspruch 1, wobei der Farbstoff ausgewählt wird aus der Gruppe bestehend aus: 9-Diethylamino-5H-benzo[alpha]phenoxazin-5-on, 1-Dimethylamino-5-sulfamoylnaphthalin, Pyren, 1-Pyrencarbaldehyd, Reichardt-Farbstoff, 4-Aminophthalimid, 4- (N,N-Dimethylamino)phthalimid, Bromnaphthalin, 2-(Dimethylamino)naphthalin und einer Kombination davon.

4. Verfahren nach Anspruch 1, wobei der Farbstoff kein N-(n-Butyl)-4-(n-butyl-amino)naphthalimid beinhaltet.

5. Verfahren nach Anspruch 1, wobei der Farbstoff ein solvatochromatischer Farbstoff ist.

6. Verfahren nach Anspruch 1, wobei das Fluid aus einer Nasspartie des Papierherstellungsverfahrens erhalten wird.

7. Verfahren nach Anspruch 1, wobei die fluorometrische Messung auf Basis von Voreinstellungen, auf intermittierender Basis und/oder kontinuierlich durchgeführt wird.

8. Verfahren nach Anspruch 1, wobei das Fluid ein wässriges Filtrat einer Pülpeaufschlämmung ist.

9. Verfahren nach Anspruch 1, wobei das Verfahren der Messung der Wirksamkeit einer oder mehrerer chemischer Substanzen dient, welche die Menge einer oder mehrerer hydrophober Verunreinigungen in einem Papierherstellungsverfahren senken, wobei das Verfahren ferner umfasst:
(i) Zugeben einer oder mehrerer chemischer Substanzen, welche die Menge der hydrophoben Verunreinigungen in dem Papierherstellungsverfahren senken, zu dem Papierherstellungsverfahren;
(ii) erneutes Messen der Menge der Verunreinigungen in dem Papierherstellungsverfahren durch Durchführen der Schritte (a) bis (f) mindestens ein weiteres Mal; und
(iii) optional Steuern der Menge der chemischen Substanzen, die dem Papierherstellungsverfahren zugesetzt werden.

10. Verfahren nach Anspruch 9, wobei es sich bei den chemischen Substanzen um mindestens eine der folgenden handelt: ein Fixiermittel; ein klebrigkeitsverringerndes Mittel; ein Dispergiermittel; ein Tensid und ein Retentionsmittel.

11. Verfahren nach Anspruch 1, wobei das Verfahren auch die Größe der hydrophoben Verunreinigungen in einem Papierherstellungsverfahren bestimmt, wobei das Verfahren ferner umfasst:
(g) mindestens einmal Laufen lassen der in Schritt (e) vermessenen Probe durch eine Einrichtung, die in der Lage ist, die Probe in eine oder mehrere wässrige Fraktionen zu teilen;
(h) mindestens einmal Messen der Fluoreszenz der wässrigen Fraktionen aus Schritt (g);
(i) Bestimmen der Größe der hydrophoben Verunreinigungen der wässrigen Fraktionen;
(j) optional Korrelieren der Fluoreszenz des Farbstoffs in den wässrigen Fraktionen mit der Konzentration der hydrophoben Verunreinigungen in den wässrigen Fraktionen; und
(k) optional Steuern der Menge der mindestens einen dem Papierherstellungsverfahren zugesetzten chemischen Substanz, welche die Verunreinigungen verringert oder inaktiviert.

12. Verfahren nach Anspruch 11, wobei die wässrigen Fraktionen einen oder mehrere suspendierte Feststoffe oder eines oder mehrere Partikel enthalten.

13. Verfahren nach Anspruch 11, wobei die Einrichtung, die eine Trennung bewirken kann, eine Zentrifuge, ein Filter oder eine Kombination davon ist.

14. Verfahren nach Anspruch 1, wobei das Verfahren auch die Größe der hydrophoben Verunreinigungen in einem Papierherstellungsverfahren bestimmt, wobei das Verfahren ferner umfasst:
(g) Filtern der in Schrtt (e) vermessenen Probe mindestens einmal durch mindestens ein Filter, wobei das Filter eine oder mehrere Poren einer bekannten Porengröße aufweist;
(h) Messen der Fluoreszenz des Filtrats der Probe aus Schritt (g) mindestens einmal;
(i) Bestimmen der Größe der hydrophoben Verunreinigungen des Filtrats und optional eines Konzentrats aus dem Filterungsschritt;
(j) optional Korrelieren der Fluoreszenz des Farbstoffs in dem Filtrat und/oder einer Absonderung mit der Konzentration der hydrophoben Verunreinigungen in dem Filtrat; und
(k) optional Steuern der Menge der mindestens einen dem Papierherstellungsverfahren zugesetzten chemischen Substanz, welche die Verunreinigungen verringert oder inaktiviert.

## Revendications

1. Procédé de surveillance d'un ou plusieurs types de contaminants hydrophobes dans un processus de fabrication du papier, comprenant:
(a) l'obtention d'un échantillon de fluide à partir dudit processus de fabrication du papier;
(b) la mesure de la turbidité de l'échantillon de fluide;
(c) la sélection d'un colorant hydrophobe qui est capable d'interagir avec lesdits contaminants présents dans ledit fluide et fluorescent dans ledit fluide;
(d) l'ajout dudit colorant audit fluide et l'octroi d'une période suffisante de temps pour que ledit colorant interagisse avec lesdits contaminants dans ledit liquide;
(e) la mesure de la fluorescence du colorant dans ledit fluide;
(f) la corrélation de la fluorescence du colorant avec la concentration desdits contaminants; et
dans lequel, si la turbidité mesurée à l'étape (b) est supérieure à 2000 NTU, ledit échantillon est dilué / davantage séparé avant l'ajout dudit colorant à l'étape (d) et la mesure de la fluorescence.

2. Procédé selon la revendication 1, dans lequel ledit fluide est filtré ou dilué ou une combinaison de ceux-ci avant ledit ajout dudit colorant ou ladite mesure de la fluorescence dudit colorant.

3. Procédé selon la revendication 1, dans lequel ledit colorant est choisi dans le groupe constitué par: le 9-diéthylamino-5H-benzo[alpha]phénoxazine-5-one, le 1-diméthylamino-5-sulfamoyl-naphtalène, le pyrène, le 1-pyrenecarbaldehyde, le colorant de Reichardt, le 4-aminophthalimide, le 4-(N,N-diméthylamino)-phthalimide, le bromonapthalène, le 2-(diméthylamino)naphtalène, et une combinaison de ceux-ci.

4. Procédé selon la revendication 1, dans lequel ledit colorant ne comprend pas de N-(n-butyl)-4-(n-butylamino)-naphtalimide.

5. Procédé selon la revendication 1, dans lequel ledit colorant est un colorant solvatochromatique.

6. Procédé selon la revendication 1, dans lequel ledit fluide est obtenu à partir d'une extrémité humide dudit processus de fabrication du papier.

7. Procédé selon la revendication 1, dans lequel ladite mesure fluorimétrique est réalisée de manière pré-établie, de manière intermittente et/ou continue.

8. Procédé selon la revendication 1, dans lequel ledit fluide est un filtrat aqueux de la pâte liquide.

9. Procédé selon la revendication 1, dans lequel le procédé est destiné à mesurer l'efficacité d'un ou plusieurs produits chimiques qui réduisent la quantité d'un ou plusieurs contaminants hydrophobes dans un processus de fabrication du papier, le procédé comprenant en outre:
(i) l'ajout d'un ou plusieurs produits chimiques audit processus de fabrication du papier qui diminuent la quantité de contaminants hydrophobes dans ledit processus de fabrication du papier;
(ii) la réévaluation de la quantité de contaminants dans ledit processus de fabrication du papier en effectuant les étapes (a) à (f) au moins une fois de plus; et
(iii) le contrôle éventuel de la quantité desdits produits chimiques qui sont ajoutés audit processus de fabrication du papier.

10. Procédé selon la revendication 9, dans lequel lesdits produits chimiques sont au moins un des éléments suivants : un fixateur ; un anti-adhésif ; un dispersant ; un tensio-actif ; et un adjuvant de rétention.

11. Procédé selon la revendication 1, dans lequel le procédé détermine également la taille desdits contaminants hydrophobes dans un processus de fabrication de papier, le procédé comprenant en outre:
(g) le passage de l'échantillon mesuré à l'étape (e) à travers un milieu capable de séparer l'échantillon en une ou plusieurs fractions aqueuses au moins une fois;
(h) la mesure de la fluorescence des fractions aqueuses provenant de l'étape (g) dudit échantillon au moins une fois;
(i) la détermination de la taille des contaminants hydrophobes des fractions aqueuses;
(j) la corrélation éventuelle de la fluorescence du colorant dans les fractions aqueuses avec la concentration desdits contaminants hydrophobes dans les fractions aqueuses; et
(k) le contrôle éventuel de la quantité d'un ou plusieurs produits chimiques qui réduisent ou inactivent lesdits contaminants qui sont ajoutés audit processus de fabrication du papier.

12. Procédé selon la revendication 11, dans lequel lesdites fractions aqueuses contiennent un ou plusieurs solides en suspension ou une ou plusieurs particules.

13. Procédé selon la revendication 11, dans lequel le moyen capable de la séparation est une centrifugeuse, un filtre ou une combinaison de ceux-ci.

14. Procédé selon la revendication 1, dans lequel le procédé détermine également la taille desdits contaminants hydrophobes dans un processus de fabrication du papier, le procédé comprenant en outre:
(g) la filtration de l'échantillon mesuré à l'étape (e) au moins une fois à travers au moins un filtre, dans lequel le filtre comprend un ou plusieurs pores ayant une taille de pore connue;
(h) la mesure de la fluorescence du filtrat provenant de l'étape (g) dudit échantillon au moins une fois;
(i) la détermination de la taille des contaminants hydrophobes du filtrat et éventuellement d'un concentré à partir de ladite étape de filtration;
(j) la corrélation éventuelle de la fluorescence du colorant dans le filtrat et/ou le rejet avec la concentration desdits contaminants hydrophobes dans le filtrat; et
(k) le contrôle éventuel de la quantité d'un ou plusieurs produits chimiques qui réduisent ou inactivent lesdits contaminants qui sont ajoutés audit processus de fabrication du papier.
